# EUROPEAN PATENT APPLICATION

(11) **EP 3 992 174 A1**
(43) Date of publication of application: **04.05.2022**
(21) Application number: 20830682.9
(22) Date of filing: 24.06.2020
(51) Int. Cl.: C07C 63/331, C07C 63/49, C07C 63/72, C07C 65/24, C07C 229/62, C07C 323/62, C07D 333/24, C07F 1/08, C07F 3/06, C07F 15/04, C01B 3/00, C07F 7/00, C07F 7/08, C07D 213/55, C07D 213/68, B01J 20/22, C07D 307/54

(54) **METAL-ORGANIC FRAMEWORK HAVING TEREPHTHALIC ACID BASED LIGAND**

(30) Priority: 25.06.2019 JP 2019117163
(71) Applicant: Rikkyo Educational Corporation, Tokyo 171-8501 (JP); Nippon Soda Co., Ltd., Tokyo 100-8165 (JP)
(72) Inventor: MINOURA, Mao, Tokyo 171-8501 (JP); SUGAMATA, Koh, Tokyo 171-8501 (JP); YANAGISAWA, Daichi, Tokyo 171-8501 (JP); KOBAYASHI, Sho, Tokyo 171-8501 (JP); IIHAMA, Teruyuki, Tokyo 100-8165 (JP)
(74) Representative: Mewburn Ellis LLP
(86) International application number: PCT/JP2020/024770
(87) International publication number: WO 2020/262451

(57) **Abstract**

Despite the fact that the amount and type of gas to be stored may vary in accordance with the type of substituent, metal-organic frameworks only using a terephthalic acid having substituents within the limited range have been produced conventionally. An object of the present invention is to provide a novel metal-organic framework using a 2,5-disubstituted terephthalic acid. A metal-organic framework comprising a carboxylate ion of formula (I) and a multivalent metal ion bound to each other is a novel metal-organic framework, enabling a gas such as hydrogen and nitrogen to be store efficiently. (wherein in formula (I),
X is an unsubstituted or substituted cycloalkyl group, an unsubstituted or substituted aryl group, an unsubstituted or substituted heterocyclyl group or -Si(R¹)(R²)(R³) ; and Y is a single bond, an alkylene group, -O-, -S-, -S(O)-, - SO₂-, -N(R⁴)- or a group formed by a combination thereof; provided that X-Y- is a phenyl group, a benzyloxy group, a pyrazol-1-yl group or a group of formula (II) except for a case where m is 3, 6, 8, 9, 10, 11 and 12).

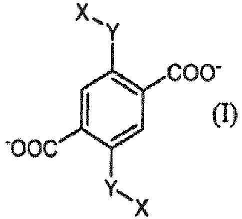

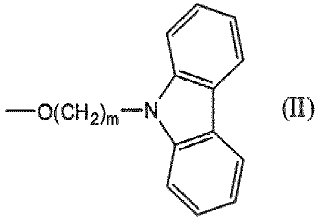

## Description

### Technical Field

The present invention relates to a novel metal-organic framework and a gas storage method using the metal-organic framework.

The present application claims priority on the basis of Japanese Patent Application No. 2019-117163 filed on June 25, 2019, the contents of which are hereby incorporated by reference.

### Background Art

A metal-organic framework (hereinafter sometimes referred to as "MOF") is a substance in a solid state that has a macromolecular structure with a space inside (that is, pores) by combining metal ions and a crosslinkable organic ligand that connects them. The metal-organic framework has been of great interest for more than a decade as a porous material with such a function as gas storage or separation.

As the crosslinkable organic ligand, an oxygen donor ligand and a nitrogen donor ligand have been often used.

MOF-5, obtainable by a Solvothermal method in which terephthalic acid is employed as a crosslinkable organic ligand and Zn(NO₃)₂•6H₂O is employed in N,N-dimethylformamide, is known to be able to store 7.1% by mass of hydrogen under conditions of temperature of 77 K and 4 MPa (see Non-patent Documents 1 to 3).

It is also known that an IRMOF (isoreticular Metal-Organic Framewark) isomorphic to MOF-5 can be provided by use of a terephthalic acid derivative having an n-propoxy group and an n-pentoxy group on the positions 2 and 5 (see Non-patent Document 2).

As examples of MOFs using a 2,5-disubstituted terephthalic acid, there are known MOFs obtainable by use of a terephthalic acid having an ω-(carbazol-9-yl)alkoxy group (the alkylene chain has 3, 6 and 8-12 carbon atoms) (see Non-patent Documents 4 and 5), an alkoxy group (6-14 carbon atoms) (see Non-patent Document 6), a methyl group, a methoxy group, a chloro group, an ethyl group and an ethoxy group (see Patent Document 1), a hydroxy group and an acetoxy group (see Non-patent Document 7), an isopropoxy group, an allyloxy group and a propargyloxy group (see Non-patent Document 8), a pyrazol-1-yl group (see Non-patent Document 9), a 4-methylbenzoyl group (see Non-patent Document 10), a benzoyl group (see Non-patent Document 11), a benzyloxy group (see Non-patent Document 12) and a bromo group (see Non-patent Document 13) and a phenyl group (see Non-patent Document 14), on the positions 2 and 5, as an organic ligand.

### Prior Art Documents

### Patent Documents

Patent Document 1: US Patent Application Publication No. 2010-75123

### Non-patent Documents

Non-patent Document 1: H. Li, M. Eddaudi, M. O'Keefe, O. M. Yaghi, Nature, 402, 276 (1999)
Non-patent Document 2: M. Eddaudi, J. Kim, N. Rosi, D. Vodak, J. Wachter, M. O'Keefe, O. M. Yaghi, Science 2002, 295 (5554), 469.
Non-patent Document 3: S. Kaye, A. Daily, O. M. Yaghi, J. Long, J. Am. Chem. Soc. 2007, 129(46), 14176.
Non-patent Document 4: E. Y. Choi, S. H. Lee, O. P. Kwon, Bull. Korean Chem. Soc. 2012, 33(7), 2431.
Non-patent Document 5: E. Y. Choi, S. B. Lee, H. Yun, S. H. Lee, C. Gao, Y. Shin, O. P. Kwon, Bull. Korean Chem. Soc. 2013, 34(12), 3903.
Non-patent Document 6: E. Y. Choi, C. Gao, H. J. Lee, O. P. Kwon, S. H. Lee, Chem. Comunn. 2009, 7563.
Non-patent Document 7: T. Yamada, H. Kitagawa, Supramolecular Chemistry 2011, 23, 315.
Non-patent Document 8: A. Schneemann, E. D. Bloch, S. Henke, P. L. Llewellyn, J. R. Long, R. A. Fischer, Eur. Chem. J. 2015, 21, 18764.
Non-patent Document 9: E. Gao, J. Xing, Y. Qu, X, Qiu, M. Zhu, Appl. Organometal Chem. 2018, 32, e4469
Non-patent Document 10: X. Zheng, S. Q. Guo, X. Y. Yu, J. K. Hu, Y. H. Luo, H. Zhang, X. Chen, Inorganic Chemistry Communication 2012, 18, 29.
Non-patent Document 11: F. Yue, B. Li, X. Zhu, Y. Luo, J. Hu, X. Wang, H. Zhang, Journal of Coordination Chemistry, 2013, 66(2), 243.
Non-patent Document 12: h. Deng, C. J. Doona, H. Furukawa, R. B. Ferreira, J. Towne, C. B. Knobler, B. Wang, O. M. Yaghi, Science 2010, 327, 846.
Non-patent Document 13: J. Gascon, M. D. Hernandez-Alonson, A. R. Almeida, G. P. M. van Klink, F. Kapteijin, G. Mui, ChemSusChem 2008, 1, 981.
Non-patent Document 14: E. R. Engel, A. Jouaiti, C. X. Bezuidenhout, M. W. Hosseini, L. J. Barbour, Angew. Chem. Int. Ed. 2017, 56, 8874.

### Summary of the Invention

### Objects to be Solved by the Invention

As described in Non-patent Document 8, despite the fact that the amount and type of gas to be stored may vary in accordance with the type of substituent, only MOFs using a terephthalic acid having substituents within the limited range described above have been produced conventionally.

An object of the present invention is to provide a novel MOF using a 2,5-disubstituted terephthalic acid.

### Means to Solve the Object

As a result of diligent studies to solve the above problems, the present inventors have found that a novel MOF can be provided by use of a combination of an unconventional 2,5-disubstituted terephthalic acid and various metal salts, and have completed the present invention.

That is, the present invention is specified by the following items that follows:
[1] A metal-organic framework comprising a carboxylate ion of formula (I) and a multivalent metal ion bound to each other: 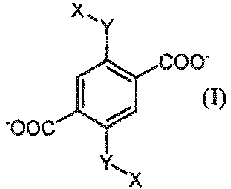 (wherein in formula (I),
   X is an unsubstituted or substituted cycloalkyl group, an unsubstituted or substituted aryl group, an unsubstituted or substituted heterocyclyl group or - Si(R¹)(R²)(R³), wherein R¹ to R³ each independently is a hydrogen atom, an unsubstituted or substituted alkyl group or an unsubstituted or substituted aryl group;
   Y is a single bond, an alkylene group, -O-, -S-, - S(O)-, -SO₂-, -N(R⁴)- or a group formed by a combination thereof; and
   R⁴ is a hydrogen atom, an unsubstituted or substituted alkyl group, an unsubstituted or substituted cycloalkyl group, an unsubstituted or substituted arylalkyl group, an unsubstituted or substituted aryl group or an unsubstituted or substituted heterocyclyl group;
   provided that X-Y- is a phenyl group, a benzyloxy group, a pyrazol-1-yl group or a group of formula (II) except for a case where m is 3, 6, 8, 9, 10, 11 and 12): 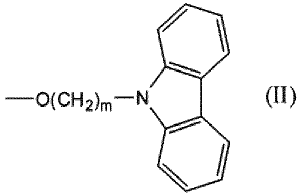
[2] The metal-organic framework according to [1], wherein the multivalent metal ion is an ion of at least one metal selected from the group consisting of Groups 2 to 13 metals in the periodic table of elements.
[3] The metal-organic framework according to [1] or [2], wherein the multivalent metal ion is an ion of at least one metal selected from Zn, Fe, Co, Ni, Cu, Al, Zr and Mg.
[4] The metal-organic framework according to any one of [1] to [3], further comprising, as a constituent, an organic ligand other than the organic ligand of formula (I) .
[5] A method for storing a gas, comprising a step of contacting a gas with the metal-organic framework according to any one of [1] to [4] to cause the gas to be adsorbed inside the metal-organic framework.

### Effect of the Invention

The MOF of the present invention is a novel MOF, which may exhibit performance in gas storage different from that of conventional MOFs.

### Mode of Carrying Out the Invention

The MOF of the present invention is an MOF comprising a carboxylate ion of formula (I) and a multivalent metal ion bound to each other.

The multivalent metal ion used in the present invention is not particularly limited as long as the multivalent metal ion is a divalent or higher metal ion. The multivalent metal ion is preferably an ion of at least one metal selected from the group consisting of Groups 2 to 13 metals in the periodic table of elements, further, preferably an ion of at least one metal ion selected from Zn, Fe, Co, Ni, Cu, Al, Zr and Mg and further preferably an ion of at least one metal selected from Co, Ni, Cu and Zn.

These multivalent metal ions are provided in the form of various salts. Examples thereof can include a nitrate, a perchlorate and a chloride ionic salt, and specific examples thereof can include Zn(NO₃)₂•6H₂O, Zn(NO₃)₂•4H₂O, Ni(NO₃)₂•6H₂O,Mg(NO₃)₂•6H₂O,Cu(NO₃)₂•xH₂O, Cu(NO₃)₂•2.5H₂O, Co(NO₃)₂•6H₂O,Al(NO₃)₃•6H₂O and ZrCl₄. Further, in consideration of the purity of the salt, ease of bonding between the metal ion and the organic ligand and the like, preferred examples thereof can include a nitrate.

The MOF of the present invention comprises a carboxylate ion of formula (I).

In formula (I), X is an unsubstituted or substituted cycloalkyl group, an unsubstituted or substituted aryl group, an unsubstituted or substituted heterocyclyl group or -Si(R¹)(R²)(R³).

As used herein, the term "unsubstituted" means that only the group of a mother nucleus is present. When only the name of the group of a mother nucleus is described without the term "substituted", it means an "unsubstituted" group unless otherwise specified.

The term "substituted" means that any hydrogen atom of the group of a mother nucleus is replaced with a functional group (substituent) having the same structure as or different structure from the structure of the mother nucleus. Therefore, the "substituent" is another functional group attached to the functional group of a mother nucleus. The substituent may be one or more. The two or more substituents are the same as or different from each other.

Such terms as "C1-6" indicate that the number of carbon atoms of the group of a mother nucleus (the functional group following the term "C1 to 6") is 1 to 6, or the like. This number of carbon atoms does not include the number of carbon atoms present in the substituent. For example, a butyl group having an ethoxy group as a substituent is classified as a C2 alkoxy-C4 alkyl group.

The "substituent" is not particularly limited as long as it is chemically acceptable and has the effect of the present invention. Examples of the group that can be a "substituent" include:
a C1-6 alkyl group such as a methyl group, an ethyl group, an n-propyl group, an i-propyl group, an n-butyl group, an s-butyl group, an i-butyl group, a t-butyl group, an n-pentyl group or an n-hexyl group;
a C2-6 alkenyl group such as a vinyl group, a 1-propenyl group, a 2-propenyl group (allyl group), a 1-butenyl group, a 2-butenyl group, a 3-butenyl group, a 1-methyl-2-propenyl group or a 2-methyl-2-propenyl group;
a C2-6 alkynyl group such as an ethynyl group, a 1-propynyl group, a 2-propynyl group (propargyl group), a 1-butynyl group, a 2-butynyl group, a 3-butynyl group or a 1-methyl-2-propynyl group;
a C3-8 cycloalkyl group such as cyclopropyl group, a cyclobutyl group, a cyclopentyl group, a cyclohexyl group or cubanyl group;
a C6-10 aryl group such as a phenyl group or a naphthyl group;
a C6 to 10 aryl-Cl to 6 alkyl group such as a benzyl group or a phenethyl group;
a 3 to 6-membered heterocyclyl group;
a 3 to 6-membered heterocyclyl-Cl to 6 alkyl group;
an oxo group;
a hydroxyl group;
a C1-6 alkoxy group such as a methoxy group, an ethoxy group, an n-propoxy group, an i-propoxy group, an n-butoxy group, an s-butoxy group, an i-butoxy group or a t-butoxy group;
a C2-6 alkenyloxy group such as a vinyloxy group, an allyloxy group, a propenyloxy group or a butenyloxy group;
a C2-6 alkynyloxy group such as an ethynyloxy group or a propargyloxy group;
a C6-10 aryloxy group such as a phenoxy group or a naphthoxy group;
a C6-10 aryl-C1-6 alkoxy group such as a benzyloxy group or a phenethyloxy group;
a 5 to 6-membered heteroaryloxy group such as a thiazolyloxy group or a pyridyloxy group;
a 5 to 6-membered heteroaryl-C1-6 alkyloxy group such as a thiazolylmethyloxy group or a pyridylmethyloxy group;
a formyl group;
a C1-6 alkylcarbonyl group such as an acetyl group or a propionyl group;
a formyloxy group;
a C1-6 alkylcarbonyloxy group such as an acetyloxy group and a propionyloxy group;
a C6-10 arylcarbonyl group such as a benzoyl group;
a C1-6 alkoxycarbonyl group such as a methoxycarbonyl group, an ethoxycarbonyl group, an n-propoxycarbonyl group, an i-propoxycarbonyl group, an n-butoxycarbonyl group or a t-butoxycarbonyl group;
a C1-6 alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group or a t-butoxycarbonyloxy group;
a carboxy group;
a halogeno group such as a fluoro group, a chloro group, a bromo group or iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group or a 2,4,6-trichlorohexyl group;
a C2-6 haloalkenyl group such as a 2-chloro-1-propenyl group or a 2-fluoro-1-butenyl group;
a C2-6 haloalkynyl group such as a 4,4-dichloro-1-butynyl group, a 4-fluoro-1-pentynyl group or a 5-bromo-2-pentynyl group;
a C1-6 haloalkoxy group such as a trifluoromethoxy group, a 2-chloro-n-propoxy group or a 2,3-dichlorobutoxy group;
a C2-6 haloalkenyloxy group such as a 2-chloropropenyloxy group or a 3-bromobutenyloxy group;
a C1-6 haloalkylcarbonyl group such as a chloroacetyl group, a trifluoroacetyl group or a trichloroacetyl group;
an amino group;
a C1-6 alkyl-substituted amino group such as a methylamino group, dimethylamino group or a diethylamino group;
a C6-10 arylamino group such as an anilino group or a naphthylamino group;
a C6-10 aryl-C1-6 alkylamino group such as a benzylamino group or a phenethylamino group;
a formylamino group;
a C1-6 alkylcarbonylamino group such as an acetylamino group, a propanoylamino group, a butyrylamino group or an i-propylcarbonylamino group;
a C1-6 alkoxycarbonylamino group such as a methoxycarbonylamino group, an ethoxycarbonylamino group, an n-propoxycarbonylamino group or an i-propoxycarbonylamino group;
a C1-6 alkylsulfoxyimino group such as a S,S-dimethylsulfoxyimino group;
an unsubstituted aminocarbonyl group or an aminocarbonyl group having a substituent, such as an aminocarbonyl group, a dimethylaminocarbonyl group, a phenylaminocarbonyl group or an N-phenyl-N-methylaminocarbonyl group;
an imino-C1-6 alkyl group such as an iminomethyl group, a 1-iminoethyl group or a 1-imino-n-propyl group;
a substituted or unsubstituted N-hydroxyimino-C1-6 alkyl group such as an N-hydroxy-iminomethyl group, a 1-(N-hydroxyimino)ethyl group, a 1-(N-hydroxyimino)propyl group, an N-methoxyiminomethyl group or a 1-(N-methoxyimino)ethyl group;
a hydroxyimino group;
a C1-6 alkoxyimino group such as a methoxyimino group, an ethoxyimino group, an n-propoxyimino group, an i-propoxyimino group or an n-butoxyimino group;
an aminocarbonyloxy group;
a C1-6 alkyl-substituted aminocarbonyloxy group such as an ethylaminocarbonyloxy group or a dimethylaminocarbonyloxy group;
a thioxo group;
a mercapto group;
a C1-6 alkylthio group such as a methylthio group, an ethylthio group, an n-propylthio group, an i-propylthio group, an n-butylthio group, an i-butylthio group, an s-butylthio group or a t-butylthio group;
a C1-6 haloalkylthio group such as a trifluoromethylthio group or a 2,2,2-trifluoroethylthio group;
a C6-10 arylthio group such as a phenylthio group or a naphthylthio group;
a 5 to 6-membered heteroarylthio group such as a thiazolylthio group or a pyridylthio group;
a C1-6 alkylsulfinyl group such as a methylsulfinyl group, an ethylsulfinyl group or a t-butylsulfinyl group;
a C1-6 haloalkylsulfinyl group such as a trifluoromethylsulfinyl group or a 2,2,2-trifluoroethylsulfinyl group;
a C6-10 arylsulfinyl group such as a phenylsulfinyl group;
a 5 to 6-membered heteroarylsulfinyl group such as a thiazolylsulfinyl group or a pyridylsulfinyl group;
a C1-6 alkylsulfonyl group such as a methylsulfonyl group, an ethylsulfonyl group or a t-butylsulfonyl group;
a C1-6 haloalkylsulfonyl group such as a trifluoromethylsulfonyl group or a 2,2,2-trifluoroethylsulfonyl group;
a C6-10 arylsulfonyl group such as a phenylsulfonyl group;
a 5 to 6-membered heteroarylsulfonyl group such as a thiazolylsulfonyl group or a pyridylsulfonyl group;
a sulfo group;
a C1-6 alkylsulfonyloxy group such as a methylsulfonyloxy group, an ethylsulfonyloxy group or a t-butylsulfonyloxy group;
a C1-6 haloalkylsulfonyloxy group such as a trifluoromethylsulfonyloxy group or a 2,2,2-trifluoroethylsulfonyloxy group;
a tri-C1-6 alkyl-substituted silyl group such as a trimethylsilyl group, a triethylsilyl group or a t-butyldimethylsilyl group;
a tri-C6-10 aryl-substituted silyl group such as a triphenylsilyl group;
a C2-C6 alkenyl-C1-C6 dialkyl-substituted silyl group such as an allyldimethylsilyl group or a vinyldimethylsilyl group;
a C1-C6 alkyl-di-C6-C10 aryl-substituted silyl group such as a t-butyldiphenylsilyl group or a diphenylmethylsilyl group;
a di-C1-C6 alkyl-C6-C10 aryl-substituted silyl group such as a dimethylphenylsilyl group;
a (C6-C10 phenyl-C1-C6 alkyl)-di-C1-C6 alkylsilyl group such a benzyldimethylsilyl group or a 3-phenylpropyldimethylsilyl group;
a C1-C6 alkyl-C6-C10 phenyl-C2-C6 alkenylsilyl group such as a methylphenylvinylsilyl group;
a tri-C1-C6 alkoxy-substituted silyl group such as a trimethoxysilyl group or a triethoxysilyl group;
a di-C1-C6 alkyl-substituted silyl group such as a dimethylsilyl group or a diethylsilyl group;
a di-C1-C6 alkoxy-substituted silyl group such as a dimethoxysilyl group or a diethoxysilyl group;
a C1-C6 alkoxy-C1-C6 alkyl-substituted silyl group such as a methoxydimethylsilyl group;
a C1-C6 alkoxy-C6-C10 aryl-substituted silyl group such as a t-butoxydiphenylsilyl group;
a C1-C6 alkyl-di-C1-C6 alkoxy-substituted silyl group such as a methyldimethoxysilyl group;
a cyano group; and
a nitro group.

In each of these "substituents", any hydrogen atom in the substituent may be also substituted with a group having a different structure. Such examples of the "substituent" can include a C1-6 alkyl group, a C1-6 haloalkyl group, a C1-6 alkoxy group, a C1-6 haloalkoxy group, a halogeno group, a cyano group or a nitro group.

The above-described "3 to 6-membered heterocyclyl group" contains, as a constituent atom of a ring, 1 to 4 heteroatoms selected from the group consisting of a nitrogen atom, an oxygen atom and a sulfur atom. The heterocyclyl group may be either monocyclic or polycyclic. As long as at least one ring in the polycyclic heterocyclyl group is a heterocyclic ring, the other ring(s) in the polycyclic heterocyclyl group may be any of a saturated alicyclic ring, an unsaturated alicyclic ring or an aromatic ring. Examples of the "3 to 6-membered heterocyclyl group" can include a 3 to 6-membered saturated heterocyclyl group, a 5 to 6-membered heteroaryl group and a 5 to 6-membered partially unsaturated heterocyclyl group.

Examples of the 3 to 6-membered saturated heterocyclyl group can include an aziridinyl group, an epoxy group, a pyrrolidinyl group, a tetrahydrofuranyl group, a thiazolidinyl group, a piperidyl group, a piperazinyl group, a morpholinyl group, a dioxolanyl group and a dioxanyl group.

Examples of the 5-membered heteroaryl group can include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group can include a pyridyl group, a pyrazinyl group, a pyrimidinyl group, a pyridazinyl group and a triazinyl group.

Examples of the 5 to 6-membered partially unsaturated heterocyclyl group can include an isoxazolinyl group and a pyrazolyl group.

Examples of the 3 to 6-membered heterocyclyl-1 to 6 alkyl group can include a glycidyl group, a 2-tetrahydrofuranylmethyl group, a 2-pyrrolylmethyl group, a 2-imidazolylmethyl group, a 3-isoxazolylmethyl group, a 5-isoxazolylmethyl group, a 2-pyridylmethyl group, a 4-pyridylmethyl group and a 3-isoxazolinylmethyl group.

In X in formula (I), examples of the "cycloalkyl group" of the "unsubstituted or substituted cycloalkyl group" can include a cyclopropyl group, a cyclobutyl group, a cyclopentyl group, a cyclohexyl group and a cubanyl group.

In X in formula (I), the "aryl group" of the "unsubstituted or substituted aryl group" may be either monocyclic or polycyclic. As long as at least one ring in the polycyclic aryl group is an aromatic ring, the other ring(s) in the polycyclic aryl group may be any of a saturated alicyclic ring, an unsaturated alicyclic ring or an aromatic ring.

Examples of the aryl group can include a phenyl group, a 1-naphthyl group, a 2-naphthyl group, an azulenyl group, an indenyl group, an indanyl group and a tetralinyl group.

In X in formula (I), examples of the "heterocyclyl group" of the "unsubstituted or substituted heterocyclyl group" can include the same as those exemplified in the "3 to 6-membered heterocyclyl group" of the "substituent".

In X in formula (I), R¹ to R³ of "-Si(R¹)(R²)(R³)" each independently is a hydrogen atom, an unsubstituted or substituted alkyl group or an unsubstituted or substituted aryl group.

The "alkyl group" of the "unsubstituted or substituted alkyl group" may be linear or branched, and examples thereof can include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methyl-n-butyl group and an i-hexyl group.

The aryl group of the "unsubstituted or substituted aryl group" may be either monocyclic or polycyclic. As long as at least one ring in the polycyclic aryl group is an aromatic ring, the other ring(s) in the polycyclic aryl group may be any of a saturated alicyclic ring, an unsaturated alicyclic ring or an aromatic ring. Examples thereof can include a phenyl group, a 1-naphthyl group, a 2-naphthyl group, an azulenyl group, an indenyl group, an indanyl group and a tetralinyl group.

Examples of "-Si(R¹)(R²)(R³)" can include a silane group, a trimethylsilyl group, a triethylsilyl group, a triisopropylsilyl group, a tri(n-butyl)silyl group, a t-butyldimethylsilyl group, a 3-cyanopropyldimethylsilyl group, a dimethyl(n-butyl)silyl group, a dimethyl(n-nonyl)silyl group, a dimethyl(n-undecanyl)silyl group, a diethylisopropylsilyl group, a dimethylisopropylsilyl group, a dimethyl(n-propyl)silyl group, a dimethyl(n-hexyl)silyl group, a dimethylstearylsilyl group, a dimethylethylsilyl group, a dimethyl(1,1,2-trimethylpropyl)silyl group, a triphenylsilyl group, a diphenylmethylsilyl group, a diphenyl(t-butyl)silyl group, a dimethyl(3-phenylpropyl)silyl group, a dimethylphenylsilyl group, a benzyldimethylsilyl group, a bromomethyldimethylsilyl group, a chloromethyldimethylsilyl group, a dimethylsilyl group, a diisopropylsilyl group and a diphenylsilyl group.

In formula (I), Y is a single bond, an alkylene group, -O-, -S-, -S(O)-, -SO₂-, -N(R⁴)- or a group formed by a combination thereof.

Examples of the alkylene group can include a methylene group, an ethylene group, a 1,3-propylene group, a 1,2-propylene group, a 2,2-propylene group, a 1,4-butylene group, a 1,3-butylene group and a 1,2-butylene group.

R⁴ is a hydrogen atom, an unsubstituted or substituted alkyl group, an unsubstituted or substituted cycloalkyl group, an unsubstituted or substituted arylalkyl group, an unsubstituted or substituted aryl group or an unsubstituted or substituted heterocyclyl group.

Examples of the alkyl group can include the same as those exemplified for R¹, examples of the cycloalkyl group, the aryl group and the heterocyclyl group can include the same as those exemplified for X, and examples of the arylalkyl group can include a benzyl group, a phenethyl group and a 3-phenylpropyl group.

As for the group formed by a combination of a single bond, an alkylene group, -O-, -S-, -S(O)-, -SO₂- and - N(R⁴)- in Y in formula (I), the combination, the number of groups to be combined and the like, as long as within a chemically acceptable range, are not particularly limited, and such a combination can made freely. Examples of the group formed by such a combination can include -NHO-, - CH₂O-, -CH₂CH₂O-, -CH₂S-, -CH₂CH₂S-, -OCH₂CH₂O-, -SCH₂CH₂S-, -OCH₂CH₂S-, -CH₂OCH₂CH₂O-, -SO₂NH- and -SO₂N(CH₃)-. The positions at which the group is bonded to the phenyl group and to X can be optionally selected within a chemically acceptable range. That is, when Y is -CH₂O-, either of the following cases is included.

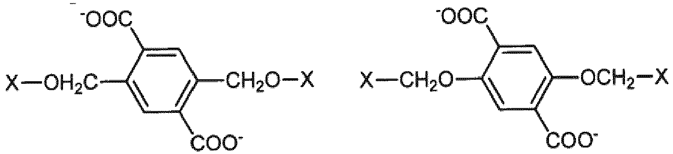

Examples of the carboxylate ion of formula (I) can include the following compounds:

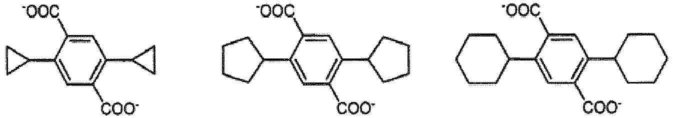

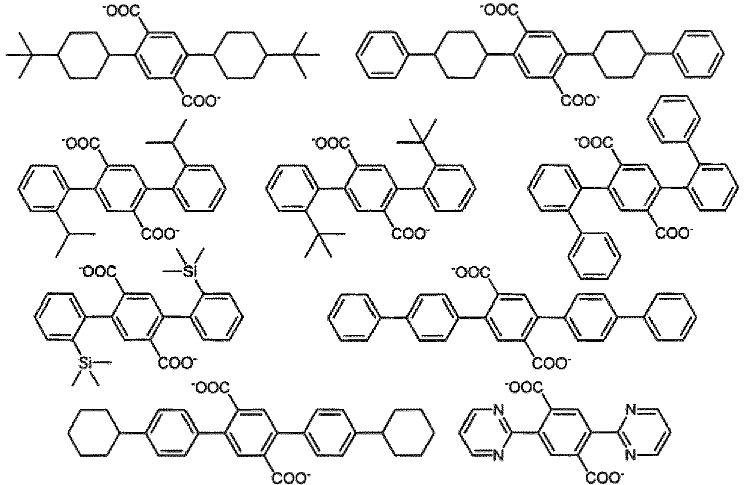

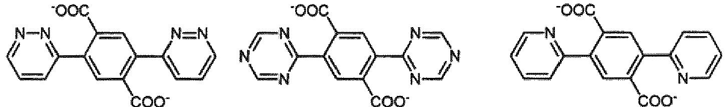

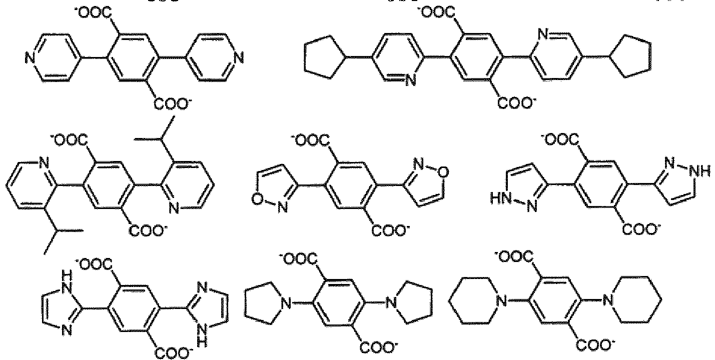

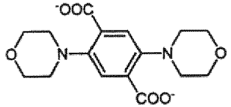

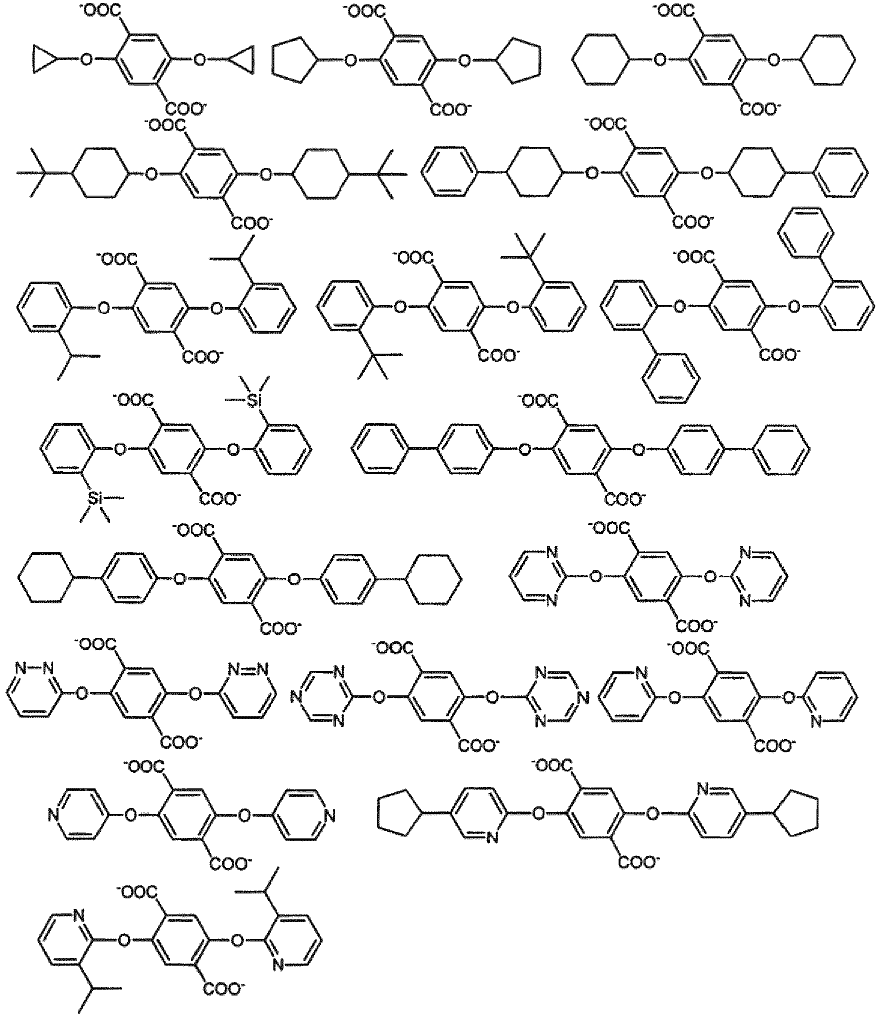

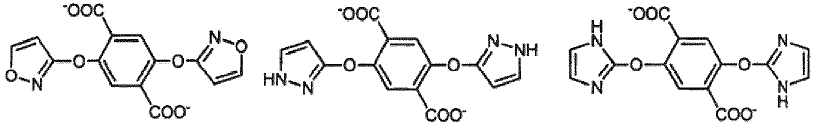

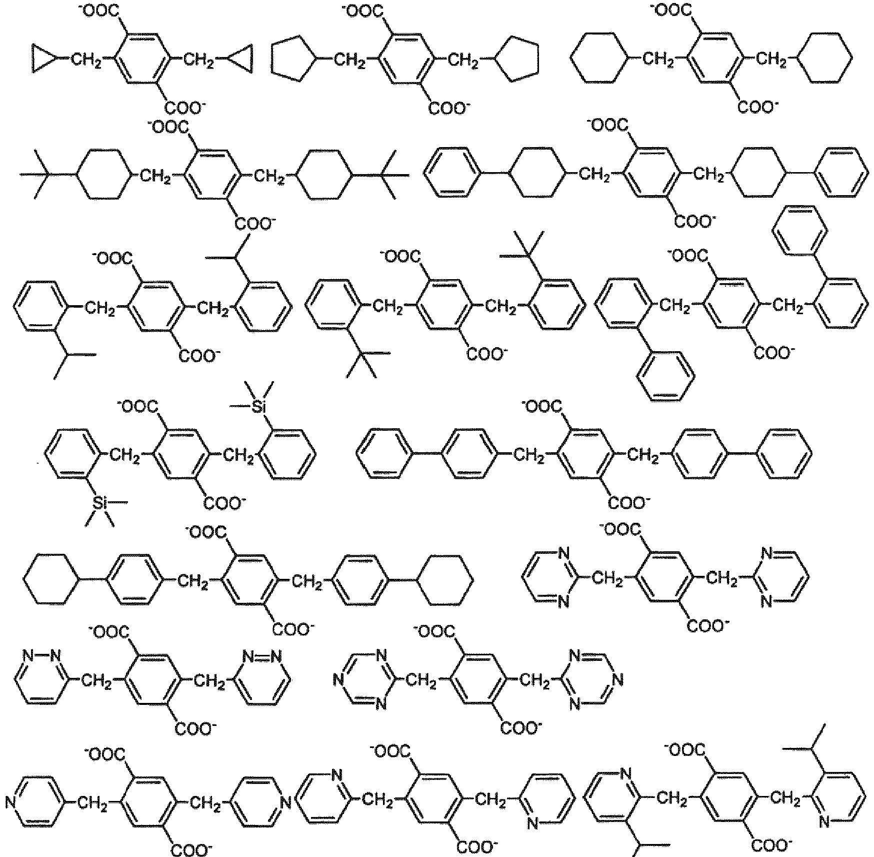

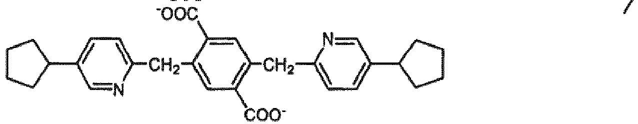

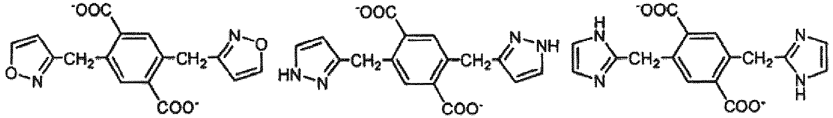

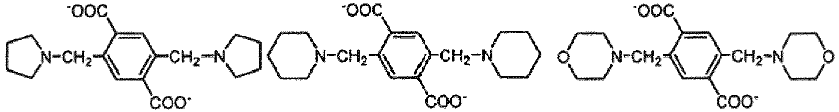

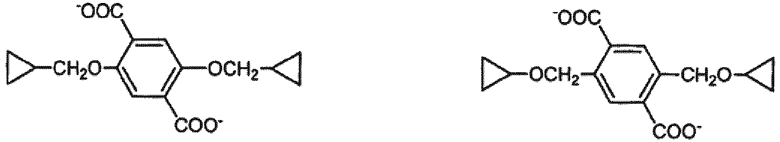

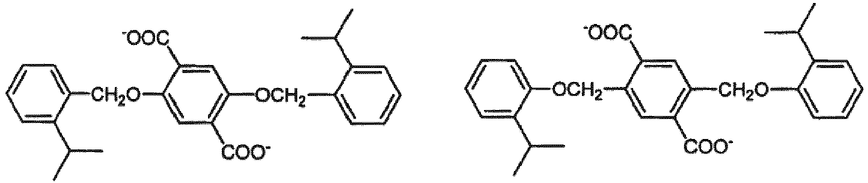

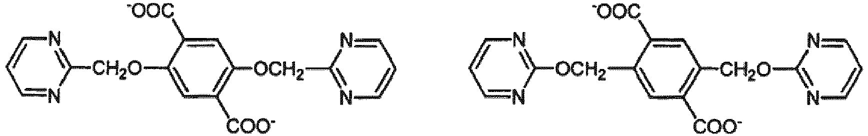

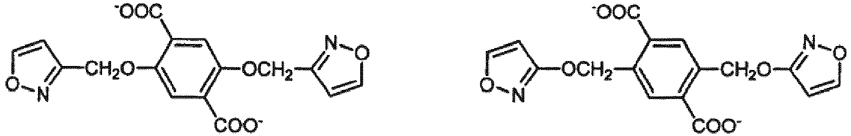

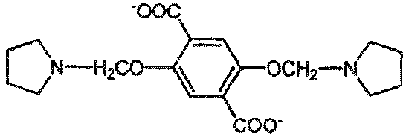

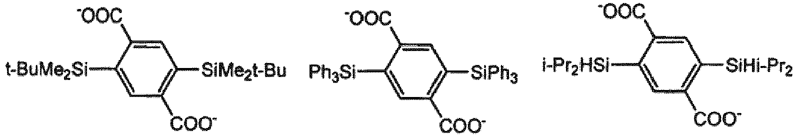

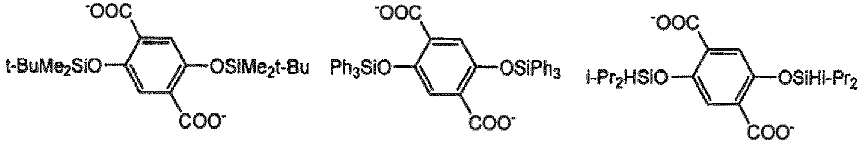

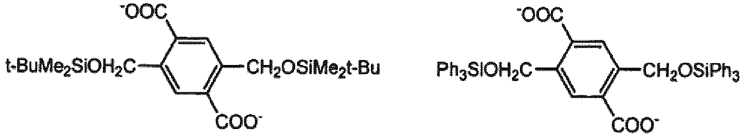

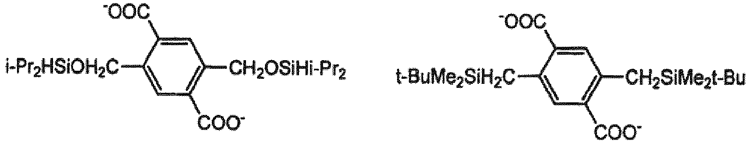

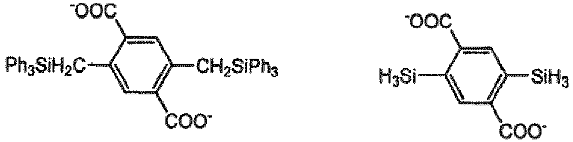

The metal-organic framework of the present invention can include an organic ligand other than the organic ligand of formula (I). Specific examples of such an organic ligand can include terephthalic acid, phthalic acid, isophthalic acid, 5-cyanoisophthalic acid, 1,3,5-trimesic acid, 1,3,5-tris(4-carboxyphenyl)benzene, 4,4'-dicarboxybiphenyl, 3,5-dicarboxypyridine, 2,3-dicarboxypyrazine, 1,3,5-tris(4-carboxyphenyl)benzene, 1,2,4,5-tetrakis(4-carboxyphenyl)benzene, 9, 10-anthracenedicarboxylic acid, 2,6-naphthalenedicarboxylic acid, [1,1':4',1"]terphenyl-3,3",5,5"-tetracarboxylic acid, biphenyl-3,3',5,5'-tetracarboxylic acid, 3,3',5,5'-tetracarboxydiphenylmethane, 1,3,5-tris(4'-carboxy[1,1'-biphenyl]-4-yl)benzene, 1,3,5-tris(4-carboxyphenyl)triazine, 1,2-bis(4-carboxy-3-nitrophenyl)ethene, 1,2-bis(4-carboxy-3-aminophenyl)ethene, trans,trans-muconic acid, fumaric acid, benzimidazole, imidazole, 1,4-diazabicyclo[2.2.2]octane (DABCO), pyrazine, 4,4'-dipyridyl, 1,2-di(4-pyridyl)ethylene, 2,7-diazapyrene, 4,4'-azobispyridine and bis(3-(4-pyridyl)-2,4-pentandionato)copper.

When the organic ligand of formula (I) and the organic ligand other than one of formula (I) are mixed and used, the mixing molar ratio is not particularly limited. However, in the case of a pillar molecule such as a pillar molecule that causes crosslinking to construct a three-dimensional structure such as a pillared-layer type structure, the organic ligand other than one of formula (I) is preferably used in an excessive amount relative to the organic ligand of formula (I).

Examples of the method for producing a metal-organic framework of the present invention that can be used include, but not limited to: a solution method such as a solvent diffusion method, a solvent agitation method or a hydrothermal method; a microwave method in which a reaction solution is irradiated with microwaves to uniformly heat the entire system in a short time; an ultrasonic method, in which a reaction vessel is irradiated with ultrasonic waves to repeatedly cause a change in pressure in the reaction vessel, leading to a phenomenon, referred to as cavitation, that a solvent forms bubbles and they collapse and in which a high energy field of about 5,000 K and 10,000 bar is locally formed and becomes a reaction field for nucleation of crystal; a solid-phase synthesis method in which a metal ion source and an organic ligand are mixed without using any solvent; and a liquid assisted grinding (LAG) method in which a metal ion source is mixed with an organic ligand with water added in an amount comparable to water of crystallization.

The method for producing a metal-organic framework of the present invention comprises, for example, steps of preparing a first solution containing a metal compound as a metal ion source and a solvent, a second solution containing a carboxylic acid as a precursor of the organic ligand of formula (I) and a solvent, and optionally, a third solution containing a compound to be another multidentate ligand and a solvent, respectively, and a step of mixing the first solution, the second solution and the third solution to prepare a reaction liquid and heating it to obtain a metal-organic framework. The first to third solutions do not need to be prepared separately. For example, the above metal compound, the carboxylic acid as the precursor of the organic ligand of formula (I), the compound to be another multidentate ligand and the solvent may be mixed at once to prepare one solution.

The mixing molar ratio of the above metal compound and the organic ligand of formula (I) can be any ratio selected depending on the pore size and surface characteristics of the metal-organic framework to be obtained, but the metal compound is preferably used in an amount of 1 mol or more and preferably used in an amount of further 1.1 mol or more, further 1.2 mol or more, further 1.5 mol or more, further 2 mol or more and further 3 mol or more relative to the organic ligand of formula (I) .

The concentration of the above metal ion in the reaction liquid is preferably in the range of 25 to 200 mmol/L.

The concentration of the organic ligand of formula (1) in the reaction liquid is preferably in the range of 10 to 100 mol/L.

The concentration of the organic ligand other than the organic ligand of formula (I) in the reaction liquid is preferably in the range of 25 to 100 mol/L.

The solvent to be used can be one or more solvent selected from the group consisting of N, N-dimethylformamide (hereinafter may be described as "DMF"), N,N-diethylformamide (hereinafter may be described as "DEF"), N,N-dimethylacetamide and water. Among them, preferred is the use of N,N-dimethylformamide, N,N-diethylformamide or dimethylacetamide alone or alternatively the use of an N,N-dimethylformamide/water-mixed solvent, an N,N-diethylformamide/water-mixed solvent or an N,N-dimethylacetamide/water-mixed solvent.

The heating temperature of the reaction liquid is not particularly limited and is preferably in the range of room temperature to 140°C.

The MOF of the present invention is a porous coordination polymer. The MOF is contemplated to be utilized, by use of the large surface area of the pores thereof, for storage of a gas and separation of a gas or as a heterogeneous catalyst, and in particular, can be employed suitably for storage of a gas. Gases that can be stored are not particularly limited. Appropriately adjusting the size of the pores enables hydrogen, nitrogen, carbon dioxide and lower hydrocarbons to be stored.

The method for storing a gas using the MOF of the present invention is, but not particularly limited to, preferably a method comprising contacting the MOF of the present invention with a gas, and the contacting manner is not particularly limited. Examples of the method include: a method in which a tank is filled with the MOF of the present invention and the gas is allowed to flow into the tank; a method in which the MOF of the present invention is supported on the surface constituting the inner wall of the tank and the gas is allowed to flow into the tank; and a method in which a tank is formed of a material containing the MOF and the gas is allowed to flow into the tank.

### Examples

Hereinafter, the present invention will be described in more detail with reference to Examples, but the present invention is not limited thereto.

The structures of carboxylic acids as the precursors of Organic ligands 1 to 31, of formula (I), used in the examples (hereinafter referred to as "Organic ligands" including the carboxylic acids as the precursors) are shown in Table 1.

Abbreviations in Table 1 denote the following meaning.

Me: methyl group, Ph: phenyl group, Py: pyridyl group, c-Pr: cyclopropyl group, c-Hex: cyclohexyl group, O: oxygen atom, N: nitrogen atom, S: sulfur atom, Cl: chlorine atom, F: fluorine atom, H: hydrogen atom, C: carbon atom.

The number in X-Y- in Table 1 represents the substitution position. For example, 2-MePh is a 2-methylphenyl group, in which, the position at which a phenyl group is bound to the benzene ring in formula (I) is taken as the position 1, and the position 2 is substituted with a methyl group.

**[Table 1]**

| 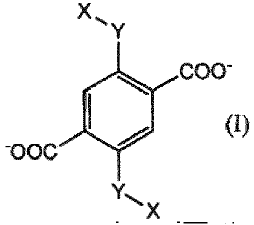 | | | |
|---|---|---|---|
| Organic ligand number | X-Y- | Organic ligand number | X - Y - |
| 1 | 2-MePh | 17 | 4-MePhO |
| 2 | 3-MePh | 18 | 4-ClPh0 |
| 3 | 4-MePh | 19 | PhNH |
| 4 | 2, 6-diMePh | 20 | Me₃Si |
| 5 | 2-ClPh | 21 | 2-naphtyl0 |
| 6 | 3-ClPh | 22 | 4-Me0Ph0 |
| 7 | 2-CF₃Ph | 23 | 3-thienyl |
| 8 | 3-CF₃Ph | 24 | 4-ClPh |
| 9 | 4-CF₃Ph | 25 | 4-MeOPh |
| 10 | 3-Py | 26 | 3-Me0Ph |
| 11 | 4-PyO | 27 | 2-Me0Ph |
| 12 | c-Pr | 28 | 3-furyl |
| 13 | c-Hex | 29 | 2-MeSPh |
| 14 | PhO | 30 | 3-MeSPh |
| 15 | 3-MePh0 | 31 | 4-MeSPh |
| 16 | 2-MePh0 | | |

### [Production Example 1] Synthesis of Organic ligand 1

Diethyl 2,5-dibromoterephthalic acid (5.0 mmol), o-methylphenylboronic acid (12.5 mmol), tetrakis(triphenylphosphine)palladium (0.05 mmol), 10 mL of a 2 M sodium carbonate aqueous solution, 35 mL of toluene and 10 mL of ethanol were heated under nitrogen at 90°C for 36 hours. It was returned to room temperature, water was added thereto, and the solution was partitioned. The organic layer was dried over magnesium sulfate and filtered. The filtrate was distilled off under reduced pressure, and the obtained solid was purified by silica gel column chromatography (chloroform). 1.5 g (3.7 mmol) of diethyl 2,5-di(o-methylphenyl)terephthalate was obtained as a yellow solid. Potassium hydroxide (100 mmol), 50 mL of ethanol and 40 mL of water were added to the obtained yellow solid and heated at 100°C for 2 hours. It was returned to room temperature, and concentrated hydrochloric acid was added thereto. It was extracted with ethyl acetate, and the organic layer was dried over magnesium sulfate and then filtered. The filtrate was distilled off under reduced pressure to obtain 1.2 g (3.46 mmol) of Organic ligand 1.

### [Production Example 2] Synthesis of Organic ligand 2

Organic ligand 2 was obtained by the same operation as in Production Example 1 except that m-methylphenylboronic acid was used instead of o-methylphenylboronic acid.

### [Production Example 3] Synthesis of Organic ligand 4

Diethyl 2,5-dibromoterephthalate (5.0 mmol), 2,6-dimethylphenylboronic acid (12.5 mmol), tris (dibenzylideneacetone)dipalladium (0.25 mmol), 2-dicyclohexylphosphino-2',6'-dimethoxybiphenyl (1.0 mmol), potassium phosphate (15 mmol) and 50 mL of toluene were refluxed under nitrogen for 2 days. It was returned to room temperature and filtered through Celite. The filtrate was distilled off under reduced pressure, and the obtained solid was purified by silica gel column chromatography (chloroform). The solid was washed with hexane and dried to obtain 2.1 mmol of diethyl 2,5-bis(2,6-dimethylphenyl)terephthalate as a colorless solid. Potassium hydroxide (40 mmol), 50 mL of ethanol and 20 mL of water were added to the obtained solid and heated at 100°C for 15 hours. It was returned to room temperature, and concentrated hydrochloric acid was added thereto. It was extracted with ethyl acetate, and the organic layer was dried over magnesium sulfate and then filtered. The filtrate was distilled off under reduced pressure to obtain 0.8 g (2.1 mmol) of Organic ligand 4.

### [Production Example 4] Synthesis of Organic ligand 5

Organic ligand 5 was obtained by the same operation as in Production Example 1 except that o-chlorophenylboronic acid was used instead of o-methylphenylboronic acid.

### [Production Example 5] Synthesis of Organic ligand 6

Organic ligand 6 was obtained by the same operation as in Production Example 1 except that m-chlorophenylboronic acid was used instead of o-methylphenylboronic acid.

### [Production Example 6] Synthesis of Organic ligand 7

Diethyl 2,5-dibromoterephthalic acid (5.0 mmol), o-trifluoromethylphenylboronic acid (12.0 mmol), [1,1'-bis(diphenylphosphino)ferrocene]palladium(II) dichloride dichloromethane adduct (0.25 mmol), potassium phosphate (20 mmol) and 20 mL of dimethoxyethane were refluxed under nitrogen for 1 day. It was returned to room temperature, washed with water, extracted with ethyl acetate, and then the organic layer was dried over magnesium sulfate. After filtration, the filtrate was distilled off under reduced pressure, and the obtained solid was purified by silica gel column chromatography (chloroform). The solid was washed with hexane and dried to obtain 2.2 mmol of diethyl 2,5-bis(o-trifluoromethylphenyl)terephthalate. Potassium hydroxide (40 mmol), 50 mL of ethanol and 20 mL of water were added to the obtained solid and heated at 100°C for 15 hours. It was returned to room temperature, and concentrated hydrochloric acid was added thereto. It was extracted with ethyl acetate, and the organic layer was dried over magnesium sulfate and then filtered. The filtrate was distilled off under reduced pressure to obtain 0.8 g (1.9 mmol) of Organic ligand 7.

### [Production Example 7] Synthesis of Organic ligand 8

Organic ligand 8 was obtained by the same operation as in Production Example 6 except that m-trifluoromethylphenylboronic acid was used instead of o-trifluoromethylphenylboronic acid.

### [Production Example 8] Synthesis of Organic ligand 9

Organic ligand 9 was obtained by the same operation as in Production Example 6 except that p-trifluoromethylphenylboronic acid was used instead of o-trifluoromethylphenylboronic acid.

### [Production Example 9] Synthesis of Organic ligand 10

2,5-Dibromo-p-xylene (5.0 mmol), 3-pyridylboronic acid (12.0 mmol), tetrakis(triphenylphosphine)palladium (0.5 mmol), sodium hydroxide (20 mmol), 20 mL of toluene, 15 mL of ethanol and 10 mL of water were refluxed under nitrogen for 1 day. It was returned to room temperature, washed with water, extracted with ethyl acetate, and then the organic layer was dried over magnesium sulfate. After filtration, the filtrate was distilled off under reduced pressure, and the obtained solid was purified by silica gel column chromatography (ethyl acetate → chloroform:methanol = 9:1). 3.4 mmol of 2,5-di(3-pyridyl)-p-xylene was obtained as a colorless solid. Potassium permanganate (20 mmol) and 15 mL of water were added to the obtained solid and heated overnight. It was returned to room temperature and filtered. Then, the residue was washed sufficiently with hot water. Hydrochloric acid was added to the filtrate to adjust the pH to 3. The filtrate was distilled off under reduced pressure, and the obtained solid was washed sufficiently with water and dried to obtain 0.3 g (0.6 mmol) of Organic ligand 10.

### [Production Example 10] Synthesis of Organic ligand 11

2,5-Dibromoterephthalic acid (10.0 mmol), 4-hydroxypyridine (40 mmol), copper powder (3.0 mmol), copper iodide (0.8 mmol), 100 mL of dimethylformamide, diazabicycloundecene (60.0 mmol) and 0.2 mL of pyridine were added and heated under reflux overnight. It was returned to room temperature, hydrochloric acid was added thereto, the precipitated solid was filtered, and the residue was washed sufficiently with water. The residue was dried to obtain 3.0 g (6.0 mmol) of Organic ligand 11 as a white solid.

### [Production Example 11] Synthesis of Organic ligand 12

Diethyl 2,5-dibromoterephthalic acid (5.0 mmol), cyclopropylboronic acid (12.0 mmol), palladium acetate (0.05 mmol), potassium phosphate (16 mmol), tricyclohexylphosphine (1.0 mmol), 20 mL of toluene and 1 mL of water was heated under nitrogen at 100°C overnight. Water was added thereto, the resultant was extracted with ethyl acetate, and the organic layer was dried over magnesium sulfate and filtered. The filtrate was distilled off under reduced pressure, and the obtained solid was purified by silica gel column chromatography (chloroform). Diethyl 2,5-dicyclopropylterephthalate was obtained as a colorless solid. Potassium hydroxide (50 mmol), 50 mL of ethanol, and 20 mL of water were added to the obtained colorless solid and heated at 100°C for 2 hours. It was returned to room temperature, and concentrated hydrochloric acid was added thereto. It was extracted with ethyl acetate, and the organic layer was dried over magnesium sulfate and then filtered. The filtrate was distilled off under reduced pressure to obtain 1.7 g (4.2 mmol) of Organic ligand 12.

### [Production Example 12] Synthesis of Organic ligand 13

Iodine (3.0 mmol) was added to p-di(cyclohexyl)benzene (30 mmol), and the resultant was dissolved in chloroform. Bromine (60 mmol) was added dropwise thereto and stirred for 48 hours. 20% sodium hydroxide was added thereto, and the resultant was extracted with diethyl ether. The extracted solution was dried over magnesium sulfate and filtered. The filtrate was distilled under reduced pressure to obtain 2,5-dibromo-1,4-dicyclohexylbenzene (27 mmol). Copper cyanide (30 mmol) was added to the obtained dibromo form (10 mmol), and the resultant was dissolved in 30 mL of dimethylformamide. The solution was refluxed overnight and returned to the room temperature. Then, 100 mL of aqueous ammonia was added thereto. The precipitated solid was filtered off, and the residue was washed sufficiently with water and dissolved in chloroform. After dried over magnesium sulfate, the solution was filtered, and the filtrate was distilled under reduced pressure. The obtained crude product was recrystallized in hexane to obtain 2,5-dicyano-1,4-dicyclohexylbenzene (9.5 mmol) as a colorless solid. A 10 M sodium hydroxide aqueous solution (75 mmol) was added to the obtained dicyano form (5.0 mmol), and the resultant was dissolved in 50 mL of ethylene glycol. Then the solution was refluxed overnight. After it was returned to the room temperature, 100 mL of water added thereto, and hydrochloric acid was added thereto to adjust the pH to pH 1. The precipitated solid was filtered off and dried sufficiently to obtain 2.2 g (4.6 mmol) of Organic ligand 13.

### [Production Example 13] Synthesis of Organic ligand 15

Organic ligand 15 was obtained by the same operation as in Production Example 10 except that m-cresol was used instead of 4-hydroxypyridine.

### [Production Example 14] Synthesis of Organic ligand 16

Organic ligand 16 was obtained by the same operation as in Production Example 10 except that o-cresol was used instead of 4-hydroxypyridine.

### [Production Example 15] Synthesis of Organic ligand 17

Organic ligand 17 was obtained by the same operation as in Production Example 10 except that p-cresol was used instead of 4-hydroxypyridine.

### [Production Example 16] Synthesis of Organic ligand 18

Organic ligand 18 was obtained by the same operation as in Production Example 10 except that p-chlorophenol was used instead of 4-hydroxypyridine.

### [Production Example 17] Synthesis of Organic ligand 21

Organic ligand 21 was obtained by the same operation as in Production Example 10 except that 2-naphthol was used instead of 4-hydroxypyridine.

### [Production Example 18] Synthesis of Organic ligand 22

Organic ligand 22 was obtained by the same operation as in Production Example 10 except that 4-methoxyphenol was used instead of 4-hydroxypyridine.

### [Production Example 19] Synthesis of Organic ligand 23

Organic ligand 23 was obtained by the same operation as in Production Example 1 except that 3-thienylboronic acid was used instead of o-methylphenylboronic acid.

### [Production Example 20] Synthesis of Organic ligand 24

Organic ligand 24 was obtained by the same operation as in Production Example 1 except that p-chlorophenylboronic acid was used instead of o-methylphenylboronic acid.

### [Production Example 21] Synthesis of Organic ligand 25

Organic ligand 25 was obtained by the same operation as in Production Example 1 except that p-methoxyphenylboronic acid was used instead of o-methylphenylboronic acid.

### [Production Example 22] Synthesis of Organic ligand 26

Organic ligand 26 was obtained by the same operation as in Production Example 1 except that m-methoxyphenylboronic acid was used instead of o-methylphenylboronic acid.

### [Production Example 23] Synthesis of Organic ligand 27

Organic ligand 27 was obtained by the same operation as in Production Example 1 except that o-methoxyphenylboronic acid was used instead of o-methylphenylboronic acid.

### [Production Example 24] Synthesis of Organic ligand 28

Organic ligand 28 was obtained by the same operation as in Production Example 1 except that 3-furylboronic acid was used instead of o-methylphenylboronic acid.

### [Production Example 25] Synthesis of Organic ligand 29

Organic ligand 29 was obtained by the same operation as in Production Example 1 except that o-methylthiophenylboronic acid was used instead of o-methylphenylboronic acid.

### [Production Example 26] Synthesis of Organic ligand 30

Organic ligand 30 was obtained by the same operation as in Production Example 1 except that m-methylthiophenylboronic acid was used instead of o-methylphenylboronic acid.

### [Production Example 27] Synthesis of Organic ligand 31

Organic ligand 31 was obtained by the same operation as in Production Example 1 except that p-methylthiophenylboronic acid was used instead of o-methylphenylboronic acid.

### [Comparative Example 1]

Terephthalic acid in an amount of 166.7 mg was dissolved in 13 mL of DMF, and 0.28 mL of triethylamine was added thereto. 17 mL of a solution of 557.7 mg of zinc acetate dihydrate in DMF was added dropwise thereto. The resultant was stirred at room temperature for 2.5 hours, and the obtained solid was separated by centrifugation. The supernatant was removed, and the solid was immersed in 20 mL of DMF overnight. Thereafter, the supernatant was removed by centrifugation, and substitution was made using chloroform. The operation of immersing the solid obtained by centrifugation in 20 mL of chloroform and centrifuging the immersed solid again was repeated three times. Thereafter, the solid obtained by centrifugation was dried under vacuum at 150°C for 5 hours to obtain 142.9 mg of Metal-organic framework A as a white powder.

### [Comparative Example 2]

DMF in an amount of 50 mL was added to 0.492 g of 2,5-dimethylterephthalic acid and 1.49 g of zinc nitrate hexahydrate and heated in an oven (reaction conditions: 120°C, 24 hours). It was returned to room temperature, and the supernatant was removed. DMF in an amount of 50 mL was added thereto, the supernatant was removed, and the solvent was replaced with chloroform. Chloroform in an amount of 50 mL was added thereto, and immersion was conducted overnight. The solid was subjected to suction filtration, and the obtained solid was dried under vacuum at 150°C for 5 hours to obtain 0.709 g of Metal-organic framework B.

### [Example 1-1]

Organic ligand 1 (0.375 mmol), zinc nitrate hexahydrate (0.375 mmol), 1,4-diazabicyclo[2.2.2]octane (0.20 mmol) and 7.5 mL of DMF were stirred for 15 minutes at room temperature and under ultrasonication. Thereafter, the filtrate obtained by centrifugation and filtration was placed in an autoclave and heated in an oven (reaction conditions: 120°C, 66 hours). It was returned to room temperature, the supernatant was discarded, and then the crystal was washed with DMF. Thereafter, the crystal was immersed in DMF overnight. Thereafter, the supernatant was removed and replaced with chloroform. Then the crystal was immersed for 1 day. The step of removing the supernatant after centrifugation was repeated three times. The solid obtained by centrifugation and removal of the supernatant was dried under vacuum at 150°C to obtain Metal-organic framework 1-1 as a white powder.

### [Example 1-2] to [Example 1-24]

Metal-organic frameworks 1-2 to 1-24 were obtained by the same operation as in Example 1-1 except that Organic ligands and solvents shown in Table 2 below were used and the reaction was conducted under the reaction conditions shown in Table 2. The results are shown in Table 2.

**[Table 2]**

| Example | Organic ligand | Solvent | Heating time (hrs.) | Property |
|---|---|---|---|---|
| 1-1 | 1 | DMF | 66 | White powder |
| 1-2 | 14 | DEF | 48 | Pale orange powder |
| 1-3 | 11 | DMF | 48 | Pale yellow powder |
| 1-4 | 5 | DMF | 48 | White powder |
| 1-5 | 6 | DMF | 48 | White powder |
| 1-6 | 18 | DMF | 185 | Pale yellow powder |
| 1-7 | 16 | DMF | 48 | Beige crystal |
| 1-8 | 15 | DMF | 48 | Bark powder |
| 1-9 | 9 | DMF | 48 | Off-white crystal |
| 1-10 | 8 | DMF | 48 | Colorless crystal |
| 1-11 | 21 | DMF | 48 | Beige powder |
| 1-12 | 22 | DMF | 48 | Gray solid |
| 1-13 | 22 | DEF | 48 | Gray solid |
| 1-14 | 25 | DMF | 49 | Colorless solid |
| 1-15 | 26 | DMF | 49 | Colorless solid |
| 1-16 | 27 | DMF | 49 | Colorless solid |
| 1-17 | 28 | DMF | 48 | Beige solid |
| 1-18 | 28 | DEF | 48 | Beige solid |
| 1-19 | 29 | DMF | 72 | Colorless solid |
| 1-20 | 29 | DEF | 72 | Brown solid |
| 1-21 | 30 | DMF | 72 | Colorless solid |
| 1-22 | 30 | DEF | 72 | Pale yellow solid |
| 1-23 | 31 | DMF | 72 | Colorless solid |
| 1-24 | 31 | DEF | 72 | Colorless solid |

### [Example 2-1]

Organic ligand 20 (0.5 mmol) was dissolved in 7 mL of DMF. A solution of zinc acetate dihydrate (1.27 mmol) in 8 mL of DMF was added dropwise thereto. The resultant was stirred at room temperature for 2.5 hours, and the obtained solid was separated by centrifugation. The supernatant was removed, and the solid was immersed in 20 mL of DMF overnight. Thereafter, the supernatant was removed by centrifugation, and substitution was made using chloroform. The washing operation of immersing the solid obtained by centrifugation in 20 mL of chloroform overnight and centrifuging the immersed solid again was repeated three times. Thereafter, the solid obtained by centrifugation was dried under vacuum at 150°C for 5 hours to obtain Metal-organic framework 2-1 as a white powder.

### [Example 2-2] to [Example 2-15]

Metal-organic frameworks 2-2 to 2-15 were obtained by the same operation as in Example 2-1 except that Organic ligands and solvents shown in Table 3 below were used and the reaction was conducted under the reaction conditions shown in Table 3. The results are shown in Table 3.

**[Table 3]**

| Example | Organic ligand | Temperature (°C) | Property |
|---|---|---|---|
| 2-1 | 20 | Room temperature | White powder |
| 2-2 | 3 | Room temperature | White powder |
| 2-3 | 12 | Room temperature | White powder |
| 2-4 | 13 | Room temperature | White powder |
| 2-5 | 11 | Room temperature | White powder |
| 2-6 | 16 | 100 | White powder |
| 2-7 | 15 | 100 | Pale yellow powder |
| 2-8 | 4 | Room temperature | White powder |
| 2-9 | 7 | Room temperature | White powder |
| 2-10 | 2 | Room temperature | White powder |
| 2-11 | 8 | Room temperature | White powder |
| 2-12 | 24 | Room temperature | Off-white powder |
| 2-13 | 25 | Room temperature | Pale yellow powder |
| 2-14 | 26 | Room temperature | Colorless powder |
| 2-15 | 27 | Room temperature | Colorless powder |

### [Example 3-1]

Organic ligand 20 (1.0 mmol) was dissolved in 13 mL of DMF, and triethylamine (0.0038 mmol) was added thereto. A solution of zinc acetate dihydrate (2.5 mmol) in 17 mL of DMF was added dropwise thereto. The resultant was stirred at room temperature for 2.5 hours, and the obtained solid was separated by centrifugation. The supernatant was removed, and the solid was immersed in 20 mL of DMF overnight. Thereafter, the supernatant was removed by centrifugation, and substitution was made using chloroform. The washing operation of immersing the solid obtained by centrifugation in 20 mL of chloroform overnight and centrifuging the immersed solid again was repeated three times. Thereafter, the solid obtained by centrifugation was dried under vacuum at 150°C for 5 hours to obtain Metal-organic framework 3-1 as a white powder.

### [Example 3-2] to [Example 3-12]

Metal-organic frameworks 3-2 to 3-12 were obtained by the same operation as in Example 3-1 except that Organic ligands and solvents shown in Table 4 below were used and the reaction was conducted under the reaction conditions shown in Table 4. The results are shown in Table 4.

**[Table 4]**

| Example | Organic ligand | Heating time (hrs.) | Property |
|---|---|---|---|
| 3-1 | 20 | 2.5 | White powder |
| 3-2 | 12 | 2.5 | White powder |
| 3-3 | 13 | 2.5 | White powder |
| 3-4 | 1 | 2.5 | White powder |
| 3-5 | 11 | 2.5 | White powder |
| 3-6 | 4 | 2.5 | White powder |
| 3-7 | 7 | 2.5 | White powder |
| 3-8 | 8 | 20 | White powder |
| 3-9 | 24 | 2.5 | Off-white powder |
| 3-10 | 25 | 2.5 | Pale yellow powder |
| 3-11 | 26 | 17 | Colorless powder |
| 3-12 | 27 | 2.5 | Colorless powder |

### [Example 4-1]

DMF in an amount of 10 mL was added to Organic ligand 14 (0.3 mmol) and zinc nitrate hexahydrate (0.4 mmol) and stirred for 5 minutes. Triethylamine (0.0068 mmol) was added dropwise thereto and stirred at room temperature for 5 minutes. After still standing for 20 minutes, the resultant was centrifuged for 10 minutes to remove the supernatant. DMF in an amount of 10 mL was added to the residue, and the resultant was centrifuged again for 10 minutes to remove the supernatant. After immersion in DMF overnight, the resultant was centrifuged for 10 minutes to remove the supernatant, and then 10 mL of chloroform was added thereto. The washing operation of immersing the solid obtained by centrifugation in 20 mL of chloroform overnight and centrifuging the immersed solid again was repeated three times. Thereafter, the solid obtained by centrifugation was dried under vacuum at 150°C for 5 hours to obtain Metal-organic framework 4-1 as a pale yellow powder.

### [Example 4-2] to [Example 4-20]

Metal-organic frameworks 4-2 to 4-20 were obtained by the same operation as in Example 4-1 except that Organic ligands shown in Table 5 below were used. The results are shown in Table 5.

**[Table 5]**

| Example | Organic ligand | Property |
|---|---|---|
| 4-1 | 14 | Pale yellow powder |
| 4-2 | 12 | White powder |
| 4-3 | 1 | White powder |
| 4-4 | 18 | Pale green powder |
| 4-5 | 5 | White powder |
| 4-6 | 6 | White powder |
| 4-7 | 16 | Pale yellow powder |
| 4-8 | 15 | Yellow powder |
| 4-9 | 3 | White powder |
| 4-10 | 20 | White powder |
| 4-11 | 17 | White powder |
| 4-12 | 2 | White powder |
| 4-13 | 9 | Pale gray powder |
| 4-14 | 8 | White powder |
| 4-15 | 21 | Green bark powder |
| 4-16 | 24 | Beige powder |
| 4-17 | 25 | Colorless powder |
| 4-18 | 26 | Colorless powder |
| 4-19 | 27 | Colorless powder |
| 4-20 | 11 | Colorless powder |

### [Example 5-1]

DMF in an amount of 3.8 mL was added to Organic ligand 14 (0.175 mmol) and zinc nitrate hexahydrate (0.352 mmol) and heated in an oven (reaction conditions: 100°C, 161 hours). It was returned to room temperature and centrifuged, and then the supernatant was removed. DMF in an amount of 10 mL was added thereto, the resultant was centrifuged, and then the solvent was removed and replaced with chloroform. Chloroform in an amount of 10 mL was added thereto, and immersion was conducted overnight. After removal of the chloroform by centrifugation, the residue was dried under vacuum at 150°C for 5 hours to obtain Metal-organic framework 5-1 as a pale orange crystal.

### [Example 5-2] to [Example 5-86]

Metal-organic frameworks 5-2 to 5-86 were obtained by the same operation as in Example 5-1 except that Organic ligands and solvents shown in Table 6 below were used and the reaction was conducted under the reaction conditions shown in Table 6. The results are shown in Table 6.

**[Table 6]**

| Example | Organic ligand | Solvent | Temperature (°C) | Heating time (hrs.) | Property |
|---|---|---|---|---|---|
| 5-1 | 14 | DMF | 100 | 161 | Pale orange crystal |
| 5-2 | 14 | DEF | 100 | 72 | |
| 5-3 | 3 | DMF | 120 | 24 | |
| 5-4 | 3 | DEF | 120 | 24 | |
| 5-5 | 19 | DMF | 120 | 24 | Bark powder |
| 5-6 | 2 | DMF | 120 | 24 | |
| 5-7 | 2 | DEF*¹ | 90 | 24 | |
| 5-8 | 2 | DEF*¹ | 120 | 24 | |
| 5-9 | 12 | DMF | 90 | 72 | Colorless crystal |
| 5-10 | 12 | DEF | 90 | 24 | Pale bark crystal |
| 5-11 | 13 | DMF | 90 | 24 | Colorless crystal |
| 5-12 | 13 | DMF | 120 | 24 | Colorless crystal |
| 5-13 | 13 | DEF | 120 | 24 | Pale bark crystal |
| 5-14 | 10 | DMF | 120 | 24 | White powder |
| 5-15 | 1 | DMF | 120 | 24 | White solid |
| 5-16 | 1 | DEF | 120 | 24 | Bark solid |
| 5-17 | 17 | DEF | 120 | 24 | Bark solid |
| 5-18 | 5 | DMF | 120 | 24 | Bark solid |
| 5-19 | 5 | DEF | 120 | 24 | Bark solid |
| 5-20 | 6 | DMF | 120 | 24 | Colorless crystal |
| 5-21 | 6 | DEF | 120 | 24 | Off-white crystal |
| 5-22 | 14 | DMF | 120 | 28 | Orange crystal |
| 5-23 | 4 | DEF | 120 | 24 | Colorless crystal |
| 5-24 | 16 | DMF | 120 | 24 | Off-white crystal |
| 5-25 | 16 | DEF | 120 | 24 | Pale bark crystal |
| 5-26 | 16 | DEF | 90 | 24 | Light gray yellow crystal |
| 5-27 | 15 | DMF | 120 | 24 | Pale bark crystal |
| 5-28 | 15 | DMF | 90 | 240 | Pale yellow crystal |
| 5-29 | 15 | DEF | 120 | 24 | Bark crystal |
| 5-30 | 15 | DEF | 90 | 140 | Light gray yellow crystal |
| 5-31 | 7 | DMF | 90 | 24 | White solid |
| 5-32 | 7 | DMF | 120 | 24 | White solid |
| 5-33 | 7 | DEF | 90 | 48 | White solid |
| 5-34 | 7 | DEF | 120 | 24 | White solid |
| 5-35 | 3 | DEF | 90 | 75 | Light gray yellow crystal |
| 5-36 | 9 | DMF | 120 | 24 | White crystal |
| 5-37 | 9 | DEF | 120 | 24 | Light gray yellow crystal |
| 5-38 | 9 | DEF | 90 | 24 | Colorless crystal |
| 5-39 | 8 | DMF | 120 | 24 | Colorless crystal |
| 5-40 | 8 | DMF | 90 | 144 | Colorless crystal |
| 5-41 | 8 | DEF | 120 | 24 | Pale yellow crystal |
| 5-42 | 8 | DEF | 90 | 72 | Colorless crystal |
| 5-43 | 21 | DEF | 120 | 48 | Bark crystal |
| 5-44 | 22 | DMF | 90 | 48 | Gray solid |
| 5-45 | 22 | DMF | 120 | 48 | Gray solid |
| 5-46 | 22 | DEF | 90 | 48 | Gray solid |
| 5-47 | 22 | DEF | 120 | 48 | Yellow bark solid |
| 5-48 | 23 | DMF | 90 | 24 | Pale yellow crystal |
| 5-49 | 23 | DMF | 120 | 24 | Colorless crystal |
| 5-50 | 23 | DEF | 90 | 24 | Yellow crystal |
| 5-51 | 23 | DEF | 120 | 24 | Orange crystal |
| 5-52 | 24 | DMF | 120 | 24 | Bark crystal + colorless solid |
| 5-53 | 24 | DMF | 90 | 24 | Colorless solid |
| 5-54 | 24 | DEF | 90 | 24 | Colorless solid |
| 5-55 | 24 | DEF | 120 | 24 | Pale bark solid |
| 5-56 | 25 | DMF | 120 | 24 | Colorless crystal |
| 5-57 | 25 | DMF | 90 | 48 | Colorless crystal |
| 5-58 | 25 | DEF | 120 | 24 | Pale yellow crystal |
| 5-59 | 25 | DEF | 90 | 48 | Off-white crystal |
| 5-60 | 26 | DMF | 120 | 24 | Colorless crystal |
| 5-61 | 26 | DMF | 90 | 48 | Colorless crystal |
| 5-62 | 26 | DEF | 120 | 24 | Pale yellow crystal |
| 5-63 | 26 | DEF | 90 | 48 | Pale yellow crystal |
| 5-64 | 27 | DMF | 120 | 24 | Colorless crystal |
| 5-65 | 27 | DMF | 90 | 48 | Colorless crystal |
| 5-66 | 27 | DEF | 120 | 24 | Beige crystal |
| 5-67 | 28 | DMF | 90 | 48 | Bark solid |
| 5-68 | 28 | DMF | 120 | 48 | Bark solid |
| 5-69 | 28 | DEF | 90 | 48 | Orange solid |
| 5-70 | 28 | DEF | 120 | 48 | Orange solid |
| 5-71 | 29 | DMF | 90 | 72 | Colorless solid |
| 5-72 | 29 | DMF | 120 | 72 | Colorless solid |
| 5-73 | 29 | DEF | 90 | 72 | Pale yellow solid |
| 5-74 | 29 | DEF | 120 | 72 | Brown solid |
| 5-75 | 30 | DMF | 90 | 72 | Colorless solid |
| 5-76 | 30 | DMF | 120 | 72 | Colorless solid |
| 5-77 | 30 | DEF | 90 | 72 | Pale brown solid |
| 5-78 | 30 | DEF | 120 | 72 | Bark solid |
| 5-79 | 31 | DMF | 90 | 72 | Pale yellow solid |
| 5-80 | 31 | DMF | 120 | 72 | Pale yellow solid |
| 5-81 | 31 | DEF | 90 | 72 | Yellow solid |
| 5-82 | 31 | DEF | 120 | 72 | Bark solid |
| 5-83 | 11 | DMF | 120 | 24 | Colorless powder |
| 5-84 | 11 | DMF | 90 | 24 | Colorless powder |
| 5-85 | 11 | DEF | 120 | 24 | Off-white powder |
| 5-86 | 11 | DEF | 90 | 24 | Off-white powder |

| | | | | | |
|---|---|---|---|---|---|
| *1: DEF was added so as to achieve 2-fold dilution in comparison with that of Example 5-1. | | | | | |

### [Example 6-1]

3 mL of a solution of Organic ligand 12 (0.38 mmol) in DMF and 2.5 mL of 1,4-diazabicyclo[2.2.2]octane (0.2 mmol) in DMF were slowly added dropwise to a solution of zinc acetate dihydrate (0.38 mmol) in 2 mL in DMF. After stirring at room temperature for 2.5 hours, the mixture was centrifuged to remove the supernatant. DMF was added thereto, and after immersion of overnight, the supernatant was removed and replaced with chloroform. Chloroform in an amount of 10 mL was added thereto, and the resultant was immersed for 1 day. The operation of centrifugation, removal of the solvent, addition of chloroform again, immersion overnight, and removal of the supernatant after centrifugation was repeated three times. The solid obtained by centrifugation and removal of the supernatant was dried under vacuum at 150°C to obtain Metal-organic framework 6-1 as a colorless crystal.

### [Example 7-1]

THF in an amount of 9 mL and water in an amount of 1 mL were added to 0.152 g of Organic ligand 25 and 0.233 g of nickel nitrate hexahydrate and heated in an oven (reaction conditions: 100°C, 48 hours). It was returned to room temperature, centrifuged and decanted to obtain a solid. The operation of adding DMF to the solid and centrifuging and decanting it was repeated three times. Then DMF was added thereto to immerse the solid overnight. The operation of removing the supernatant, adding chloroform to the solid, and centrifuging and decanting it was repeated three times. The solvent was replaced with chloroform, and chloroform was added thereto to immerse the solid overnight. The solid obtained by decanting the solid was dried under vacuum at 150°C for 5 hours to obtain 0.178 g of Metal-organic framework 7-1.

### [Example 7-2] to [Example 7-17]

Metal-organic frameworks 7-2 to 7-17 were obtained in the same operation as in Example 4-1 except that Organic ligands shown in Table 7 below and the reaction was conducted under the reaction conditions shown in Table 7. The results are shown in Table 7.

**[Table 7]**

| Example | Organic ligand | Heating time (hrs.) | Property |
|---|---|---|---|
| 7-1 | 25 | 48 | Brown bark crystal |
| 7-2 | 26 | 48 | Pale green + ocherous powder |
| 7-3 | 27 | 48 | Yellow bark crystal |
| 7-4 | 14 | 24 | Bark solid |
| 7-5 | 11 | 24 | Pale green crystal |
| 7-6 | 3 | 48 | Pale bark solid |
| 7-7 | 9 | 48 | Brown powder |
| 7-8 | 13 | 48 | Yellow bark powder |
| 7-9 | 18 | 48 | Bark powder |
| 7-10 | 2 | 48 | Red bark powder |
| 7-11 | 6 | 48 | Beige powder |
| 7-12 | 5 | 48 | Yellow powder |
| 7-13 | 16 | 48 | Bark powder |
| 7-14 | 15 | 49 | Bark powder |
| 7-15 | 17 | 49 | Bark powder |
| 7-16 | 8 | 48 | Pale green + ocherous powder |
| 7-17 | 1 | 48 | Red bark powder |

### [Example 8-1]

0.114 g of Organic ligand 25, 0.0702 g of zirconium chloride, 0.0625 g of water, 0.5406 g of acetic acid and 4 mL of DMF were stirred for 5 minutes at room temperature and under ultrasonication. Thereafter, the mixture was heated in an oven (reaction conditions: 120°C, 24 hours). It was returned to room temperature, centrifuged and decanted to obtain a solid. The operation of adding DMF to the solid and centrifuging and decanting it was repeated three times. The supernatant was removed, and DMF was added thereto to immerse the solid overnight. The operation of decanting the immersed solid after centrifugation, adding acetone to the obtained solid, and centrifuging and decanting it was repeated three times. The supernatant was removed, and acetone was added thereto to immerse the solid overnight. The solid obtained by centrifugation and decantation was dried under vacuum at 150°C for 5 hours to obtain 0.0826 g of Metal-organic framework 8-1.

### [Example 8-2] to [Example 8-10]

Metal-organic frameworks 8-2 to 8-10 were obtained by the same operation as in Example 8-1 except that Organic ligands shown in Table 8 below were used. The results are shown in Table 8.

**[Table 8]**

| Example | Organic ligand | Property |
|---|---|---|
| 8-1 | 25 | Colorless powder |
| 8-2 | 26 | White powder |
| 8-3 | 14 | Yellow solid |
| 8-4 | 18 | Bark solid |
| 8-5 | 3 | Colorless powder |
| 8-6 | 16 | Pale yellow solid |
| 8-7 | 15 | Pale yellow powder |
| 8-8 | 17 | Beige solid |
| 8-9 | 2 | Colorless powder |
| 8-10 | 8 | Colorless powder |

### [Example 9-1]

Organic ligand 25 in an amount of 0.0759 g, 0.0484 g of copper nitrate trihydrate and 4 mL of DMF were added and stirred, and then the mixture was filtered. Thereafter, the mixture was heated in an oven (reaction conditions: 120°C, 24 hours). It was returned to room temperature, centrifuged and decanted to obtain a solid. The operation of adding DMF to the solid and centrifuging and decanting it was repeated three times. Then DMF was added thereto, and the solid was immersed overnight. The supernatant was removed, and the solvent was replaced with chloroform. The operation of addition of chloroform, washing and filtration under pressure was repeated three times, and chloroform was added thereto to immerse the solid overnight. The solid was subjected to filtration under pressure, and the obtained solid was dried under vacuum at 150°C for 5 hours to obtain 0.0670 g of Metal-organic framework 9-1.

### [Example 9-2] to [Example 9-32]

Metal-organic frameworks 9-2 to 9-32 were obtained by the same operation as in Example 9-1 except that Organic ligands shown in Table 9 below were used. The results are shown in Table 9.

**[Table 9]**

| Example | Organic ligand | Heating time (hrs.) | Heating temperature (°C) | Solvent | Property |
|---|---|---|---|---|---|
| 9-1 | 25 | 24 | 120 | DMF | Light blue powder |
| 9-2 | 26 | 24 | 120 | DMF | Light blue powder |
| 9-3 | 27 | 24 | 120 | DMF | Light blue powder |
| 9-4 | 14 | 24 | 120 | DMF | Emerald green crystal |
| 9-5 | 14 | 24 | 90 | DMF | Emerald green crystal |
| 9-6 | 14 | 24 | 120 | DEF | Green crystal |
| 9-7 | 14 | 24 | 90 | DEF | Light blue solid |
| 9-8 | 11 | 24 | 120 | DMF | Light blue solid |
| 9-9 | 11 | 24 | 90 | DMF | Light blue solid |
| 9-10 | 11 | 24 | 120 | DEF | Light blue + yellow green solid |
| 9-11 | 11 | 24 | 90 | DEF | Colorless solid |
| 9-12 | 1 | 24 | 120 | DMF | Blue solid |
| 9-13 | 1 | 24 | 90 | DMF | Light blue solid |
| 9-14 | 1 | 24 | 120 | DEF | Blue solid |
| 9-15 | 1 | 24 | 90 | DEF | Light blue solid |
| 9-16 | 3 | 43 | 120 | DMF | Light blue solid |
| 9-17 | 3 | 43 | 90 | DMF | Light blue solid |
| 9-18 | 3 | 43 | 120 | DEF | Light blue solid |
| 9-19 | 3 | 43 | 90 | DEF | Light blue solid |
| 9-20 | 2 | 24 | 120 | DMF | Light blue solid |
| 9-21 | 2 | 24 | 90 | DMF | Light blue solid |
| 9-22 | 2 | 24 | 120 | DEF | Light blue solid |
| 9-23 | 2 | 24 | 90 | DEF | Light blue solid |
| 9-24 | 9 | 48 | 120 | DMF | Dark blue powder |
| 9-25 | 9 | 48 | 90 | DMF | Light blue powder |
| 9-26 | 9 | 48 | 120 | DEF | Dark blue powder |
| 9-27 | 9 | 48 | 90 | DEF | Dark light blue solid |
| 9-28 | 13 | 40 | 120 | DMF | Purple powder |
| 9-29 | 16 | 24 | 120 | DMF | Dark blue powder |
| 9-30 | 15 | 24 | 120 | DMF | Dark blue powder |
| 9-31 | 18 | 40 | 120 | DMF | Light blue powder |
| 9-32 | 11 | 0.5 | RT | DMF | Colorless powder |

### [Examples 10-1 and 10-2]

Organic ligand 11 in an amount of 0.1839 g, 0.0521 g of copper acetate monohydrate, 0.1098 g of acetic acid and 10 mL of DMF were added to a vial and ultrasonicated for 5 minutes. Thereafter, the mixture was heated in an oven (reaction conditions: 70°C, 7 days). When the mixture was returned to room temperature, light blue and white solids were obtained. The solids were visually separated and centrifuged, and then decanted to obtain each solid. DMF was added to each solid to immerse each solid overnight. DMF was removed by centrifugation and decantation, and the solvent was replaced with chloroform. For the blue solid, this step was repeated three times. The obtained solid was dried under vacuum at 150°C for 5 hours to obtain 0.0538 g of Metal-organic framework 10-1. For the white solid, the operation of centrifugation and filtration under pressure was repeated three times. Then, the obtained solid was dried under vacuum at 150°C for 5 hours to obtain 0.0258 g of Metal-organic framework 10-2.

### (Measurement of BET specific surface area and measurement of hydrogen storage capacity)

The BET specific surface area and the hydrogen storage capacity at 77 K and atmospheric pressure were measured for some of Metal-organic frameworks obtained. The hydrogen storage amount at 298 K and 10 MPa was also measured for some of Metal-organic frameworks.

The BET specific surface area and the hydrogen storage capacity at 77 K and atmospheric pressure were measured with a gas adsorption measurement device, Tristar-II (manufactured by Micromeritics Instrument Corporation)

The BET specific surface area was calculated according to the following procedure. About 50 mg of each of Metal-organic frameworks was placed inside a glass cell. The pressure inside the glass cell was reduced to vacuum at a temperature of 135°C and the inside of the glass cell was dried for 6 hours. The glass cell was attached to the gas adsorption measurement device and immersed in a temperature-controlled bath containing liquid nitrogen. The pressure of nitrogen contained in the glass cell was gradually increased. The measurement was carried out until the pressure of nitrogen introduced into the glass cell reached 1.0 × 10⁵ Pa.

The hydrogen storage capacity at 77K and a normal pressure was calculated according to the following procedure. After the measurement for nitrogen, the gas type was changed to hydrogen to carry out the measurement. The pressure of hydrogen contained in the glass cell was gradually increased. The measurement was carried out until the pressure of hydrogen introduced into the glass cell reached 1.0 × 10⁵ Pa.

The results of the BET specific surface areas measured were shown in Table 10.

The hydrogen storage capacities measured at 77 K and atmospheric pressure are shown in Table 11.

**[Table 10]**

| Metal-organic framework number | BET specific surface area (m²/g) | Metal-organic framework number | BET specific surface area (m²/g) | Metal-organic framework number | BET specific surface area (m²/g) |
|---|---|---|---|---|---|
| 1-9 | 601 | 5-4 | 1028 | 5-66 | 907 |
| 2-1 | 733 | 5-6 | 560 | 5-72 | 945 |
| 2-2 | 530 | 5-7 | 555 | 8-1 | 511 |
| 2-3 | 1187 | 5-8 | 655 | 8-2 | 442 |
| 2-4 | 616 | 5-9 | 1659 | 8-3 | 411 |
| 2-5 | 1939 | 5-10 | 1591 | 8-4 | 419 |
| 2-6 | 810 | 5-11 | 1013 | 8-5 | 591 |
| 2-7 | 755 | 5-12 | 807 | 8-8 | 466 |
| 2-8 | 522 | 5-13 | 712 | 8-9 | 665 |
| 2-9 | 531 | 5-15 | 990 | 8-10 | 679 |
| 2-10 | 653 | 5-16 | 984 | B | 426 |
| 2-11 | 445 | 5-17 | 448 | | |
| 2-14 | 653 | 5-18 | 771 | | |
| 3-1 | 870 | 5-19 | 895 | | |
| 3-2 | 563 | 5-20 | 720 | | |
| 3-3 | 700 | 5-21 | 846 | | |
| 3-4 | 481 | 5-22 | 982 | | |
| 3-5 | 2019 | 5-23 | 786 | | |
| 3-6 | 486 | 5-24 | 586 | | |
| 3-7 | 551 | 5-25 | 606 | | |
| 3-8 | 533 | 5-26 | 884 | | |
| 3-12 | 449 | 5-27 | 872 | | |
| 4-1 | 963 | 5-28 | 909 | | |
| 4-2 | 1322 | 5-29 | 752 | | |
| 4-3 | 1057 | 5-30 | 538 | | |
| 4-5 | 851 | 5-31 | 729 | | |
| 4-6 | 773 | 5-32 | 743 | | |
| 4-7 | 580 | 5-33 | 824 | | |
| 4-8 | 597 | 5-34 | 714 | | |
| 4-9 | 437 | 5-35 | 1063 | | |
| 4-10 | 961 | 5-36 | 1294 | | |
| 4-11 | 483 | 5-37 | 851 | | |
| 4-12 | 706 | 5-38 | 746 | | |
| 4-13 | 830 | 5-39 | 697 | | |
| 4-14 | 637 | 5-41 | 972 | | |
| 4-17 | 690 | 5-42 | 753 | | |
| 4-18 | 656 | 5-59 | 723 | | |
| 4-19 | 606 | 5-60 | 545 | | |
| 5-1 | 1107 | 5-62 | 670 | | |
| 5-2 | 991 | 5-64 | 921 | | |
| 5-3 | 874 | 5-65 | 889 | | |

**[Table 11]**

| Metal-organic framework number | Hydrogen storage capacity (wt%) | Metal-organic framework number | Hydrogen storage capacity (wt%) |
|---|---|---|---|
| 2-3 | 1. 108 | 5-13 | 1. 177 |
| 2-5 | 1. 564 | 5-15 | 1.284 |
| 2-6 | 1. 149 | 5-16 | 1. 267 |
| 2-7 | 1. 042 | 5-18 | 1.043 |
| 2-14 | 1. 018 | 5-19 | 1. 158 |
| 3-3 | 1.071 | 5-21 | 1. 137 |
| 3-5 | 1. 676 | 5-22 | 1. 151 |
| 4-1 | 1. 122 | 5-26 | 1. 188 |
| 4-2 | 1. 262 | 5-27 | 1. 162 |
| 4-3 | 1. 394 | 5-28 | 1. 161 |
| 4-5 | 1. 116 | 5-29 | 1. 028 |
| 4-6 | 1. 021 | 5-35 | 1. 393 |
| 4-17 | 1. 130 | 5-40 | 1. 226 |
| 4-18 | 1.068 | 5-41 | 1. 337 |
| 5-1 | 1. 242 | 5-42 | 1.530 |
| 5-2 | 1. 072 | 5-59 | 1. 265 |
| 5-3 | 1. 298 | 5-62 | 1. 076 |
| 5-4 | 1. 496 | 5-64 | 1. 337 |
| 5-9 | 1. 541 | 5-65 | 1. 306 |
| 5-10 | 1. 486 | 5-66 | 1. 316 |
| 5-11 | 1. 568 | 5-72 | 1. 060 |
| 5-12 | 1. 283 | A | 1. 111 |

### Industrial Applicability

The metal-organic framework of the present invention can store gases such as hydrogen and nitrogen at a practical level. Consequently, the metal-organic framework can make hydrogen to be utilized more easily, toward the advent of a hydrogen energy-based society.

## Claims

1. A metal-organic framework comprising a carboxylate ion of formula (I) and a multivalent metal ion bound to each other: 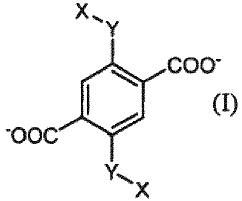 (wherein in formula (I),
X is
an unsubstituted or substituted cycloalkyl group,
an unsubstituted or substituted aryl group,
an unsubstituted or substituted heterocyclyl group or -Si(R¹)(R²)(R³);
wherein R¹ to R³ each independently is
a hydrogen atom,
an unsubstituted or substituted alkyl group or an unsubstituted or substituted aryl group;
Y is
a single bond,
an alkylene group,
-O-,
-S-,
-S(O)-,
-SO₂-,
-N(R⁴)-
or a group formed by a combination thereof; and
R⁴ is
a hydrogen atom,
an unsubstituted or substituted alkyl group,
an unsubstituted or substituted cycloalkyl group,
an unsubstituted or substituted arylalkyl group,
an unsubstituted or substituted aryl group
or an unsubstituted or substituted heterocyclyl group;
provided that
X-Y- is a phenyl group, a benzyloxy group, a pyrazol-1-yl group or a group of formula (II) except for a case where m is 3, 6, 8, 9, 10, 11 and 12):

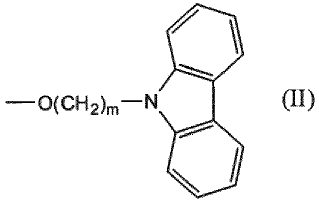

2. The metal-organic framework according to claim 1, wherein the multivalent metal ion is an ion of at least one metal selected from the group consisting of Groups 2 to 13 metals in the periodic table of elements.

3. The metal-organic framework according to claim 1 or 2, wherein the multivalent metal ion is an ion of at least one metal selected from Zn, Fe, Co, Ni, Cu, Al, Zr and Mg.

4. The metal-organic framework according to any one of claims 1 to 3, further comprising, as a constituent, an organic ligand other than an organic ligand of formula (I).

5. A method for storing a gas, comprising a step of contacting a gas with the metal-organic framework according to any one of claims 1 to 4 to cause the gas to be adsorbed inside the metal-organic framework.
